(19)

Europäisches
Patentamt
European
Patent Office
Office européen
des brevets

(11) **EP 2 550 918 A1**

(12) **EUROPEAN PATENT APPLICATION**
published in accordance with Art. 153(4) EPC

(43) Date of publication:
**30.01.2013 Bulletin 2013/05**

(21) Application number: **11759201.4**

(22) Date of filing: **09.03.2011**

(51) Int Cl.:
*A61B 5/18* (2006.01)    *B60K 28/06* (2006.01)
*B60R 11/04* (2006.01)    *B60W 40/08* (2012.01)
*G08G 1/16* (2006.01)

(86) International application number:
**PCT/JP2011/055547**

(87) International publication number:
**WO 2011/118393 (29.09.2011 Gazette 2011/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2010 JP 2010067001**

(71) Applicant: **Aisin Seiki Kabushiki Kaisha Aichi 448-8650 (JP)**

(72) Inventors:
• **KADOYA, Akira**
  **Kariya-shi, Aichi-ken**
  **448-8650 (JP)**
• **KAWANO, Takashi**
  **Kariya-shi, Aichi-ken**
  **448-8650 (JP)**

(74) Representative: **TBK**
  **Bavariaring 4-6**
  **80336 München (DE)**

(54) **ALERTNESS DETERMINATION DEVICE, ALERTNESS DETERMINATION METHOD, AND PROGRAM**

(57) A reference value for detecting the initial state before a predetermined time elapses is set immediately after the vehicle begins to operate. The reference value functions as a relatively accurate indicator for indicating that the driver is highly alert. Thus, it is possible to accurately determine whether or not a driver is highly alert by comparing the aforementioned reference value with the blink frequency, which functions as an indicator for reflecting the current state of the driver.

FIG.1

EP 2 550 918 A1

## Description

Technical Field

The present invention relates to an alertness determination device, an alertness determination method and program, and more specifically to an alertness determination device for determining a driver's alertness, and an alertness determination method and program for determining a driver's alertness.

In recent years the number of traffic accidents has remained at a high level despite a downward trend in the number of people killed in such accidents. There are various causes for accidents, but one factor that induces accidents is a driver operating a vehicle while in a state of reduced alertness.

Hence, various devices have been proposed for avoiding traffic accidents by emitting a warning when the driver's alertness drops (for example, see Patent Literature 1). The device disclosed in Patent Literature 1 detects an openness value indicating the openness of the driver's eyes from images of the driver, and collects these chronologically. In addition, the device finds the statistical representative value and statistical deviation of the openness value. The statistical representative value expresses the magnitude of the openness value and the statistical deviation is the scattering of an openness value equivalent to blink frequency.

Next, this device creates a determination standard for determining from the statistical representative value and the statistical deviation whether the driver's eyes are open or closed. Furthermore, the determination standard and the openness value are compared, and a determination as to whether or not the driver's alertness has dropped is made based on the ratio of the time the eyes are closed to a unit time.

Prior Art Literature

Patent Literature

Patent Literature 1: Unexamined Japanese Patent Application Kokai Publication No. 2006-109980.

Disclosure of Invention

Problems to be Solved by the Invention

A driver operating an automobile is not always looking forward, as at times the driver may glance sideways or look downward in order to confirm gauges and/or the like. Consequently, an eye openness value detected based on images from a camera will tend to be less than the actual value. In this case, there are concerns that accuracy in detecting alertness could drop.

In addition, a driver's blink frequency declines at a point in time shortly after the alertness has begun to drop, and the blink frequency increases immediately before the driver's alertness begins to drop or when the alertness has begun to drop. Accordingly, it is possible to accurately detect the initial state of alertness beginning to drop by determining alertness based on blink frequency.

In consideration of the foregoing, it is an objective of the present invention to accurately detect the initial state of a driver that appears when alertness begins to drop.

Means for Solving the Problems

In order to achieve the above objective, an alertness determination device according to a first aspect of the present invention is an alertness determination device for determining the alertness of a driver driving a vehicle, this alertness determination device comprising:

a first count means for counting the frequency with which the driver blinks based on images obtained by photographing the driver, when the driver is in a prescribed state;
a reference value setting means for setting a reference value based on the count results of the first count means;
a second count means for counting the frequency with which the driver blinks within a prescribed time based on images obtained by photographing the driver, after the reference value is set; and
a determination means for determining that the alertness of the driver is declining when the frequency counted by the second count means exceeds the reference value.

An alertness determination method according to a second aspect of the present invention is an alertness determination method for determining the alertness of a driver driving a vehicle, this alertness determination method comprising:

a first procedure for counting the frequency with which the driver blinks based on images obtained by photographing the driver, when the driver is in a prescribed state;
a second procedure for setting a reference value based on the count results of the first procedure;
a third procedure for counting the frequency with which the driver blinks within a prescribed time based on images obtained by photographing the driver, after the reference value is set; and
a fourth procedure for determining that the alertness of the driver is declining when the frequency counted in the third procedure exceeds the reference value.

A program according a third aspect of the present invention is a program for causing a computer to function as:

a first count means for counting the frequency with which the driver blinks based on images obtained by photographing the driver, when the driver is in a prescribed state;

a reference value setting means for setting a reference value based on the count results of the first count means;

a second count means for counting the frequency with which the driver blinks within a prescribed time based on images obtained by photographing the driver, after the reference value is set; and

a determination means for determining that the alertness of the driver is declining when the frequency counted by the second count means exceeds the reference value.

Efficacy of the Invention

With the present invention, it is possible to accurately detect the initial state of a driver that appears when alertness begins to drop, based on changes in blink frequency.

Brief Description of Drawings

FIG. 1 is a block diagram of an alertness determination system according to a first preferred embodiment of the present invention;

FIG. 2 is a drawing showing one example of an image output from a photography device;

FIG. 3 is a block diagram of an alertness determination system according to a second preferred embodiment of the present invention;

FIG. 4 is a flowchart for explaining the actions of the alertness determination device;

FIG. 5 is a drawing for explaining the relationship between driver blinking and facial orientation; and

FIG. 6 is a flowchart for explaining the actions of an alertness determination device according to a third preferred embodiment of the present invention.

Mode for Carrying Out the Invention

(First Preferred Embodiment)

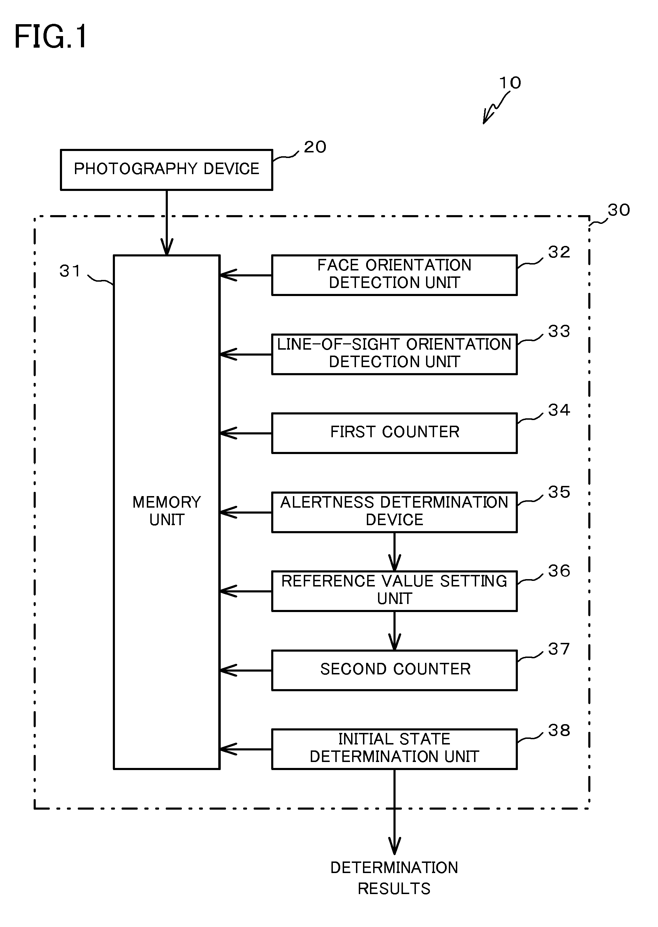

Below, a first preferred embodiment of the present invention is described with reference to the drawings. FIG. 1 is a block diagram showing a summary composition of an alertness determination system 10 according to the first preferred embodiment. The alertness determination system 10 is for example mounted in an automobile and determines whether or not a driver is in an alert state based on images of the driver output from a photography device 20.

This alertness determination system 10 has the photography device 20 and an alertness determination device 30 for determining whether or not the driver is in an alert state, based on images output from the photography device 20, as shown in FIG. 1.

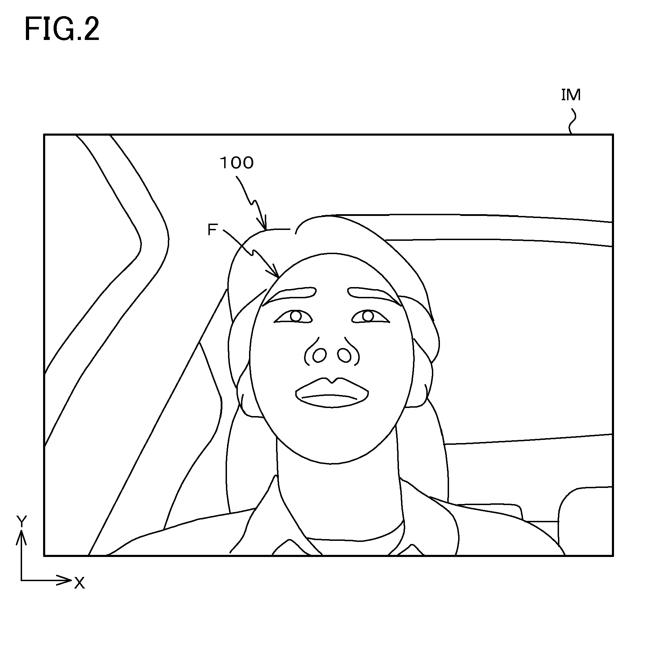

The photography device 20 is a device for outputting images acquired by photographing a subject, after converting such into an electrical signal. FIG. 2 is a drawing showing one example of an image output from the photography device. As can be seen by referring to an image IM shown in FIG. 2, this photography device 20 is attached for example on a vehicle's windshield, dashboard or steering column cover so that the face F of the driver 100 seated in the vehicle's driver's seat is positioned in the center of the field of vision. Furthermore, this device successively photographs the driver 100 and successively sends image information D(m) relating to images of the driver 100 to the alertness determination device 30.

Here, m is an integer 1 or larger and indicates the photography frame number of the image. For example, the image information D(m) is image information obtained through the mth photograph. In addition, the value of m is reset when the vehicle's ignition switch is turned on by the driver 100. In other words, the value m is reset when driving by the driver 100 is started.

Returning to FIG. 1, the alertness determination device 30 has a memory unit 31, a face orientation detection unit 32, a line-of-sight orientation detection unit 33, a first counter 34, an alertness determination unit 35, a reference value setting unit 36, a second counter 37 and an initial state determination unit 38.

The memory unit 31 chronologically stores image information successively output from the photography device 20 and in addition successively stores information relating to process results from the aforementioned units 32 to 38.

The face orientation detection unit 32 detects changes in the orientation of the face of the driver 100 with reference to the image information D(m) photographed while a time H1 elapses, for each time H1 (for example 10 minutes) that elapses from a time t1 when the vehicle's ignition switch is turned on.

For example, the face orientation determination unit 32 executes an imaging process using a Sobel filter on each image specified by image information D(m) output during the period from the time t1 until the time H1 has elapsed, and detects the outline of the driver's face and the outline of the eyelids. Furthermore, this unit detects the orientation of the face of the driver 100 as a vector $A_m(n)$ from the positional relationship between the outline of the face and the outline of the eyelids. Next, the face orientation detection unit 32 detects the time at which an angle between the vector $A_m(n)$ specified based on the image information D(m) and a vector $A_{m+1}(n)$ specified based on image information D(m+1) is at least a prescribed angle. Furthermore, time information TA(m) related to this time is stored in the memory unit 31. After this, the face orientation detection unit 32 repeats the above-described actions for each time H1 that elapses.

The line-of-sight orientation detection unit 33 detects changes in the orientation of the line of sight of the driver 100 by referencing the image information D(m) photographed while the time H1 was elapsing, for each time H1 that elapses from the time t1.

For example, the line-of-sight orientation detection unit 33 executes an image process using a Sobel filter for each of the images specified by the image information D(m) output during the interval from the time t1 until the time H1 has elapsed, and detects the outline of the driver's eyelids and the outline of the iris between those eyelids. Furthermore, this device detects the orientation of the line of sight of the driver 100 as a vector $B_m(n)$ from the positional relationship between the eyelid outline and the iris outline. Next, the face orientation detection unit 32 detects the time at which an angle between the vector $B_m(n)$ specified based on the image information D(m) and a vector $B_m(n)$ specified based on image information D(m+1) is at least a prescribed angle. Furthermore, time information TB(m) related to this time is stored in the memory unit 31. After this, the face orientation detection unit 32 repeats the above-described actions for each time H1 that elapses.

The first counter 34 counts the frequency C1(i) with which the driver 100 blinks, with reference to the image information D(m) photographed while the time H1 elapses, for each time H1 that elapses, from the time t1. Here, i is an integer 1 or greater.

For example, the counter 34 detects the outline of the driver's eyelids by executing an image process using a Sobel filter for images specified by the image information D(m). Furthermore, blinks by the driver 100 are detected from the shape of the outline of the eyelids, and the frequency C1(i) with which blinks are detected is counted. When detecting the frequency C1, the first counter 34 references the time information TA(m) and the time information TB(m) and does not count the frequency C1 when blinks are detected based on the image information D(m) related to the time information TA(m) and the time information TB(m). In other words, the first counter 34 counts the frequency C1 of blinks in the interval during which the time H1 elapses, excluding blinks detected when the face orientation of the driver has changed and blinks detected when the orientation of the driver's line of sight has changed.

The first counter 34 successively stores information related to the frequency C1(i) in the memory unit 31. Below, the first counter 34 repeats the above-described actions for each time H1 that elapses. Through this, information relating to a frequency C1(1), a frequency C1(2), ..., and a frequency C1(i) is successively stored in the memory unit 31.

The alertness determination unit 35 first makes a determination about the alertness of the driver 100 based on information related to the frequency C1(1) of blinks stored in the memory unit 31. Specifically, the alertness determination unit 35 compares the frequency C1(1) and a prescribed threshold value and determines whether or not the frequency C1(1) is less than the prescribed threshold value.

Furthermore, the alertness determination unit 35 determines that the driver is not in an alert state when the frequency C1(1) is less than the threshold value. In this case, the alertness determination unit 35 again executes a determination of the alertness of the driver 100 based on information related to the frequency C1(2). Following this, the alertness determination unit 35 repeats determinations of alertness until the determination based on the frequency C1(i) is affirmed or the frequency of these determinations reaches a prescribed frequency (for example, five times).

Furthermore, when the frequency C1(i) is at least as great as the threshold value, the alertness determination unit 35 notifies the reference value setting unit 36 that the driver 100 is in an alert state. On the other hand, when the frequency C1(i) is not at least as great as the threshold value even once as a result of executing determinations with a prescribed frequency based on information related to the frequency C1(i), the alertness determination unit 35 notifies the reference value setting unit 36 that the driver 100 is not in an alert state.

The reference value setting unit 36 sets a reference value for detecting the initial state when alertness drops, in accordance with notification contents from the alertness determination unit 35. For example, when notification is received that the driver 100 is in an alert state, the reference value setting unit 36 sets the newest frequency C1(i) as the reference value S. On the other hand, when notification is received that the driver 100 is not in an alert state, the reference value setting unit 36 sets the average value of the frequencies C1(1), C1(2), ..., C1(i) as the reference value S. Furthermore, the reference value setting unit 36 stores information relating to the reference value S in the memory unit 31 and notifies the second counter 37 that setting of the reference value S has concluded.

Upon being notified that the reference value has been set, the second counter 37 counts the frequency C2(j) with which the driver 100 blinks, with reference to the image information D(m) photographed while a time H2 elapses, for each time H2 (for example 5 minutes) that elapses, from the time t2 that the reference value was set. Here, j is an integer 1 or greater. Furthermore, the second counter 37 successively stores information relating to the frequency C2(j) in the memory unit 31. After this, the second counter 37 repeats the above-described actions for each time H2 that elapses. Through this, information relating to the frequencies C2(1), C2(2),..., C2(j) is successively stored in the memory unit 31.

The initial state determination unit 38 successively compares the reference value S and the frequency C2(j). Furthermore, the initial state determination unit 38 determines that the state of the driver 100 is an initial state when alertness is dropping when the frequency C2(j) is larger than a value found by adding a correction value $\alpha$ to the reference value S, and outputs the determination results to an external device and/or the like. This external device acts based on the determination results. For example, this external device could emit a warning to the driver 100 or could cause the brakes to be automatically applied.

As described above, with this preferred embodiment a reference value S is set for detecting the initial state when a prescribed time has elapsed since the time t1 when the vehicle's ignition switch was turned on, based on photographed images of the driver. In general, the driver is in a state of high alertness immediately after the driver begins driving. Consequently, the reference value S to a certain degree becomes an indicator accurately showing that the driver is in a state of high alertness.

With this preferred embodiment, a determination is made as to whether or not the driver's state is an initial state when alertness drops by comparing this reference value S to a blink frequency that is an indicator reflecting the current state of the driver. Accordingly, whether or not the driver is in a state of high alertness can be determined with good accuracy.

With this preferred embodiment, blinks detected when the orientation of the face of the driver 100 has changed and blinks detected when the orientation of the line of sight of the driver 100 has changed are not counted by the first counter 34. Consequently, for example when the driver 100 looks downward without blinking, even though the angle between the driver's eyelids and the photography device 20 changes, the first counter 34 does not erroneously recognize this change as a blink by the driver 100, and this change is not counted as blink frequency. Accordingly, the reliability of the reference value S set based on the blink frequency increases, and as a result whether or not the driver is in a state of high alertness can be accurately determined.

With this preferred embodiment, whether or not the alertness of the driver is high is determined when the blink frequency of the driver in each time interval H2 is greater than the reference value S. Accordingly, the initial state when the alertness of the driver drops can be detected with good accuracy.

(Second Preferred Embodiment)

Next, a second preferred embodiment of the present invention is described with reference to the drawings. Compositions that are the same or equivalent to those of the first preferred embodiment are labeled using the same symbols and explanation of such is abbreviated or omitted.

The alertness determination system 10 according to this preferred embodiment differs from the alertness determination system 10 according to the first preferred embodiment in that the alertness determination device 30 is realized by a typical computer or a composition similar to a device such as a microcomputer and/or the like.

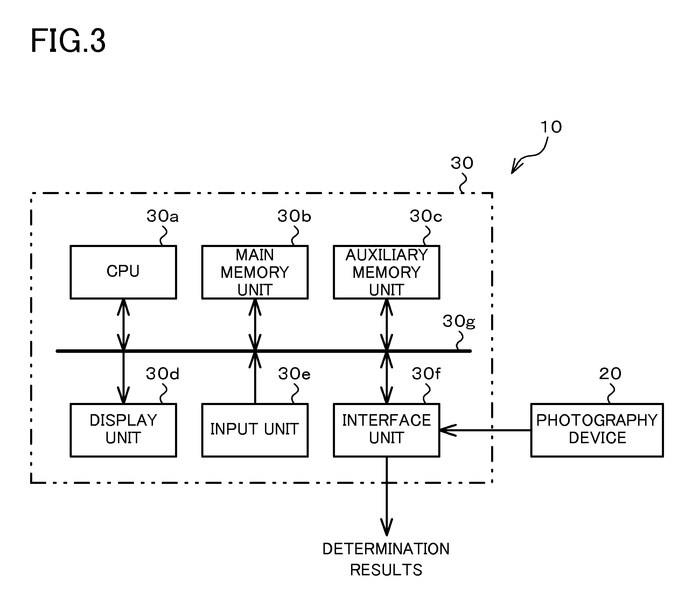

FIG. 3 is a block diagram showing the physical composition of the alertness determination system. As shown in FIG. 3, the alertness determination system 10 is composed of the photography device 20 and the alertness determination device 30 composed of a computer.

The alertness determination device 30 includes a CPU (Central Processing Unit) 30a, a main memory unit 30b, an auxiliary memory unit 30c, a display unit 30d, an input unit 30e, an interface unit 30f and a system bus 30g for mutually connecting the above-described units.

The CPU 30a executes the below-described processes on image information D(m) output from the photography device 20 in accordance with a program stored in the auxiliary memory unit 30c.

The main memory unit 30b has a composition including RAM (Random Access Memory) and/or the like, and is used as a work area for the CPU 30a.

The auxiliary memory unit 30c has a composition including non-volatile memory such as a ROM (Read Only Memory), magnetic disk and/or semiconductor memory. This auxiliary memory unit 30c stores programs executed by the CPU 30a and various types of parameters. In addition, this unit successively stores information relating to images output from the photography device 20 and information including process results from the CPU 30a and/or the like.

The display unit 30d is composed of a CRT (Cathode Ray Tube), or an LCD (Liquid Crystal Display) and/or the like, and displays process results from the CPU 30a.

The input unit 30e is composed of a keyboard and a pointing device such as a mouse and/or the like. Instructions from the operator are input via this input unit 30e and are sent to the CPU 30a via the system bus 30g.

The interface unit 30f is composed of a serial interface or a LAN (Local Area Network) interface and/or the like. The photography device 20 is connected to the system bus 30g via the interface unit 30f.

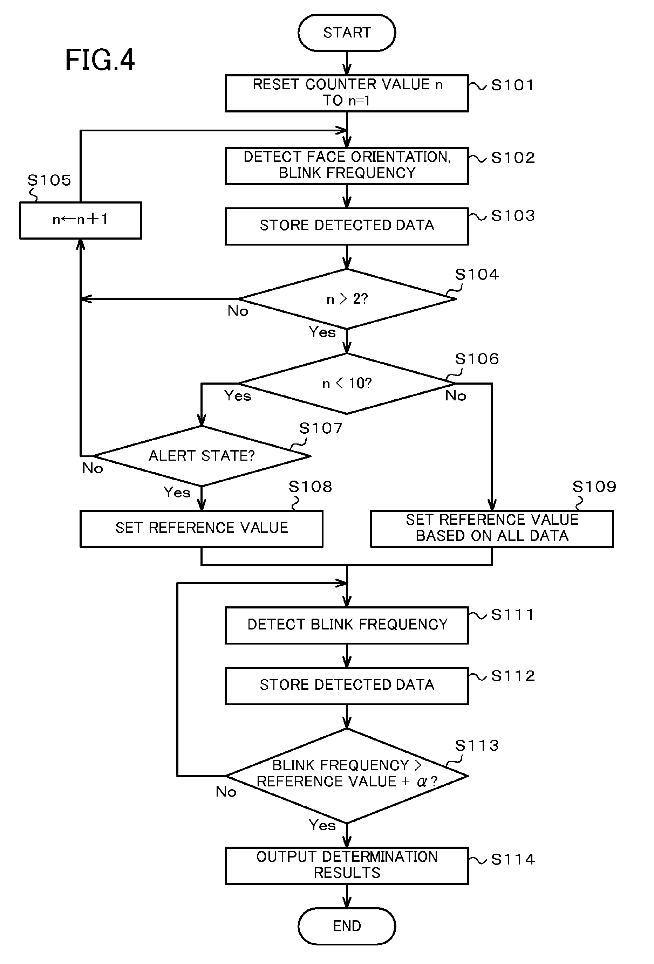

The flowchart in FIG. 4 corresponds to a series of process algorithms of a program executed by the CPU 30a. Below, the process executed by the alertness determination device 30 is explained with reference to FIG. 4.

The alertness determination system 10 is started when the vehicle's ignition switch is turned on by the driver 100. The process shown in FIG. 4 is executed after information relating to images photographed by the photography device 20 are output from the photography device 20.

First, in the initial step S101, the CPU 30a resets the count number n to 1.

Next, in step S102, the CPU 30a detects the facial orientation of the driver and the blink frequency of the driver when facing forward.

For example, the CPU 30a executes an imaging process using a Sobel filter on images specified by the image information D(m) output during the interval from the time t1 when output from the photography device 20 started until the time H1 (for example 10 minutes) has elapsed, and detects the outline of the driver's face and the outline of the eyelids. Here, m is an integer 1 or greater.

Next, the CPU 30a detects the orientation of the face of the driver 100 as the vector $A_m(n)$ from the positional relationship of the facial outline and the eyelid outline, for each piece of image information D(m). Furthermore, when it is determined that the driver 100 is facing in the direction in which the vehicle is moving based on the vector $A_m(n)$, the frequency C1(n) with which the driver 100 blinks is detected. The frequency C1(n) is specifically detected from chronological changes in the outline of the eyelids photographed in images specified by the image information D(m). The CPU 30a accomplishes detection of the frequency C1(n) with the time H1 as one unit.

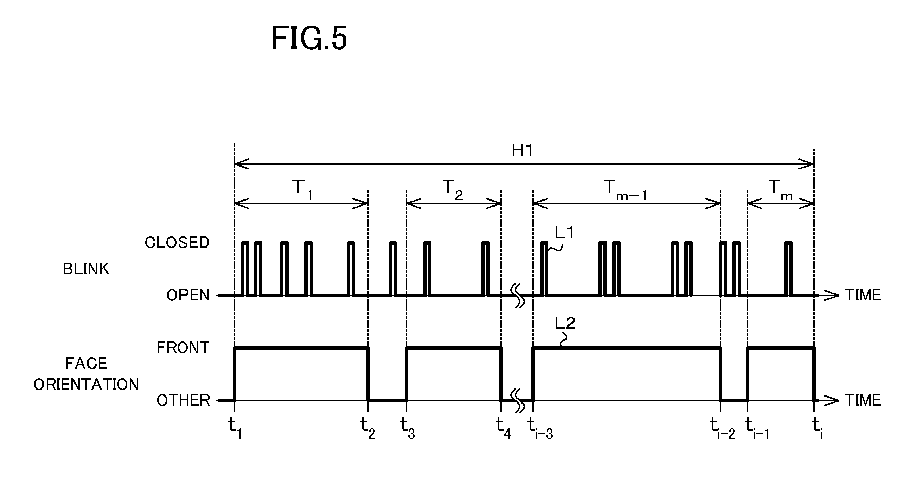

FIG. 5 is a drawing for explaining the relationship between the facial orientation of and blinking by the driver 100. The solid line L1 in FIG. 5 is at a high level when the eyes of the driver 100 are closed, and is at a low level when the eyes are open. In addition, the solid line L2 is at a high level when the facial orientation of the driver 100 is toward the front (the direction in which the vehicle is traveling), and is at a low level when oriented in any other direction.

For example, when opening and closing of the eyes and changes in the facial orientation are changed as shown by solid lines L1 and L2 in FIG. 5, the CPU 30a first finds the frequency $c_m$ of rises in the solid line L1 during time spans in which the solid line L2 is at a high level. For example, during the interval from time $t_1$ to time $t_2$ when the solid line L1 is at a high level, the value of the frequency $c_1$ is 5. In addition, during the interval from time $t_3$ to time $t_4$ when the solid line L1 is at a high level, the value of the frequency $c_2$ is 2. Furthermore, the CPU 30a computes the frequency C1(n) as the sum of the frequencies $c_m$ based on equation (1).

$$C(n) = c_1 + c_2 + \ldots + c_m \qquad (1)$$

Next, the CPU 30a computes the time T(n) during which the solid line L2 was at a high level while the time H1 was elapsing based on equation (2).

$$T(n) = T_1 + T_2 + \ldots + T_m \qquad (2)$$

Next, the CPU 30a divides the frequency C(n) by the time T(n) to find the blink frequency per unit time k1(n) (= C(n) / T(n)).

Furthermore, the CPU 30a in the next step S103 stores information relating to the vector $A_m(n)$ detected for each piece of image information D(m), and information relating to the frequency k1(n), in the auxiliary memory unit 30c.

Next, in step S104, a determination is made as to whether or not the counter value n is larger than 2. When the determination in step S104 is negative, the CPU 30a moves to the next step S105 and increments the counter value n by 1, after which the CPU returns to step S102. From here, the CPU 30a repeatedly executes the process from step S102 to step S105 until the determination in step S104 is affirmative.

Through this, information relating to the vectors $A_m(1)$, $A_m(2)$ and $A_m(3)$, and information relating to the blink frequencies per unit time k1(1), k1(2) and k1(3) are stored in the auxiliary memory unit 30c each time the time H1 elapses.

The alertness determination system 10 starts when the automobile's ignition switch is turned on. Consequently, with the alertness determination device 30, it can be conceived that for a little while after the system is started, various information is detected from images when the driver is for example looking at the surroundings of the vehicle, setting the seat position or checking gauges. In this case, the information detected by the alertness determination device 30 becomes unstable. The process in this step S 104 is executed with the intent of avoiding processes starting from step S106 being performed on the basis of this unstable information.

On the other hand, when the determination in step S 104 is affirmative, the CPU 30a moves to step S106.

In step S106, the CPU 30a determines whether or not the counter value n is less than 10. When the determination in step S106 is affirmative, the CPU 30a moves to step S107.

In step S107, the CPU 30a detects the frequency D with which the facial orientation of the driver 100 changed while the time H1 was elapsing based on information relating to the newest vector $A_m(n)$ out of the vectors $A_m(1)$, $A_m(2)$, $A_m(3)$, ... $A_m(n)$ showing the facial orientation of the driver 100. Specifically, the CPU 30a detects the frequency with which the facial orientation changes to an orientation other than forward, in other words the frequency with which the solid line L2 rises, as the frequency D.

Furthermore, the frequency D and the threshold value are compared, and when the frequency D is at least as great as the threshold vale, the driver 100 is determined to be in an alert state (step S107: Yes).

When the determination in step S107 is affirmative, the CPU 30a moves to step S108. Furthermore, the CPU 30a sets the recently detected frequency k1(n) as the reference value S.

On the other hand, when the determination in step S 107 is negative, the CPU 30a increments the counter value n by 1 in step S105 and then returns to step S102. Following this, the CPU 30a repeatedly executes the processes from steps S102 to S107.

When the processes from steps S102 to S107 are repeatedly executed, eventually the counter value n becomes larger than 10 and the determination in step S106 is negative. In this case, the CPU 30a moves to step S109. When the CPU 30a moves to step S109, information relating to the blink frequencies per unit time k1(1), k1(2), ..., k1(10) are stored in the auxiliary memory unit 30c.

In step S109, the CPU 30a sets the average value $k_{AVG}$ of the frequencies k1(3), k1(4),...,k1(10) as the reference value S.

The process in step S109 is executed when the determination is step S107 is negative and the reference value S has not been set in step S108 even when a prescribed time has elapsed. In this case, the reference value S is set based on information acquired to that point by the process in step S109 being executed.

In the next step S111, the CPU 30a executes the same process as in step S 102 and successively detects the blink frequency per unit time k2(j) when the driver 100 is facing forward.

In the next step S112, the CPU 30a stores information relating to the frequency k2(j) in the auxiliary memory unit 30c.

In the next step S113, the CPU 30a determines whether or not the frequency k2(j) is larger than a value found by adding the correction value α to the reference value S. When the determination in step S113 is negative (step S113: No), the CPU 30a returns to step S111. Furthermore, the CPU 30a repeatedly executes steps S111 through S113 until the determination in step S113 is affirmative.

On the other hand, when the determination in step S113 is affirmative (step S113: Yes), the CPU 30a moves to step S114.

In step S114, the CPU 30a determines that the status of the driver 100 is an initial state when alertness is declining, and outputs the determination results to an external device and/or the like.

As explained above, with this preferred embodiment while a prescribed time is elapsing from a time t1 when the vehicle's ignition switch is turned on, a reference value S for detecting an initial state is set. Consequently, this reference value S becomes an indicator accurately indicating to a certain degree that the driver is in a state of high alertness.

With this preferred embodiment, the reference value S is set based on the blink frequency when the driver is facing forward. Consequently, for example if the driver 100 looks downward without blinking, even though the angle between the driver's eyelids and the photography device 20 changes, this change is not recognized erroneously as a blink by the driver 100 and is thus not counted as blink frequency. Accordingly, the reliability of the reference value S set based on the blink frequency is further increased.

With this preferred embodiment, by comparing this reference value S and the blink frequency k2(j) that is an indicator reflecting the driver's current status, a determination is made as to whether or not the driver is in a state of high alertness. Accordingly, it is possible to accurately determine whether or not the driver is in a state of high alertness.

With this preferred embodiment, the driver's alertness is determined to be high when the driver's blink frequency per unit time k2(j) exceeds the reference value S. Accordingly, it is possible to accurately detect the initial state when the driver's alertness declines.

(Third Preferred Embodiment)

Next, a third preferred embodiment of the present invention will be described with reference to the drawings. Compositions that are the same as or comparable to those of the second preferred embodiment are labeled with the same reference numbers, and explanation of such is abbreviated or omitted.

The alertness determination system 10 according to this preferred embodiment differs from the alertness determination system 10 according to the second preferred embodiment in that the processes corresponding to steps S 106 to S108 are not executed by the CPU 30a, and in that the contents of the process corresponding to step S102 differ.

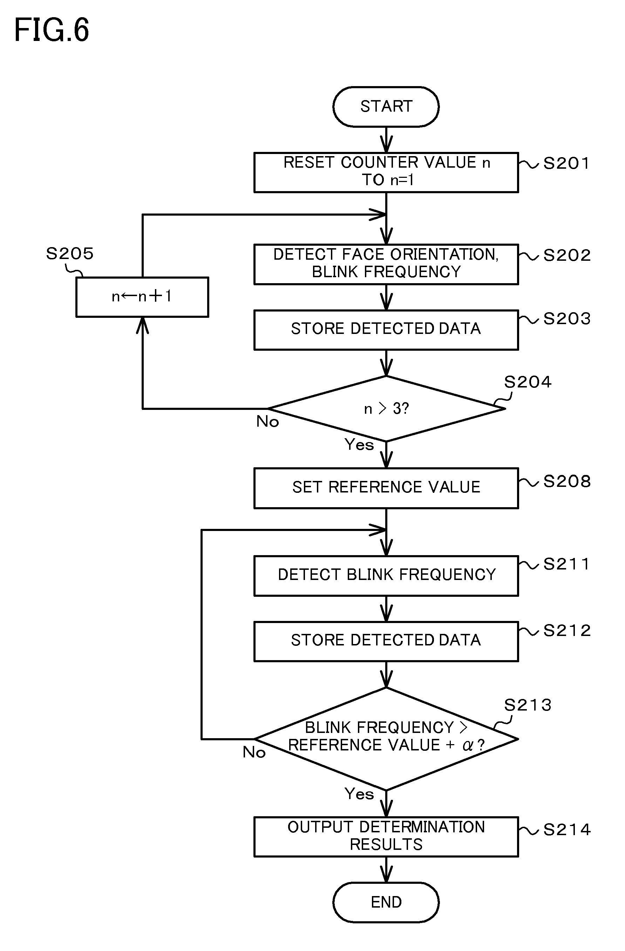

The flowchart in FIG. 6 corresponds to a series of process algorithms of programs executed by the CPU 30a. Below, the actions of the alertness determination device 30 according to this preferred embodiment are explained with reference to FIG. 6.

First, in the initial step S201, the CPU 30a resets the counter value n to 1.

In the next step S202, the CPU 30a detects the frequency with which the driver blinks. For example, the CPU 30a executes image processing using a Sobel filter and detects the outlines of the driver's eyelids for each image specified by the image information D(m) output during the interval from the time t1 when output from the photography device 20 starts until a time H1 (for example, 10 minutes) has elapsed.

Next, the CPU 30a detects the frequency C1(n) with which the driver 100 blinks from changes with time in the outline of the eyelids shown in images specified by the image information D(m). Furthermore, the blink frequency per unit time k1(n) is computed by dividing the frequency C1(n) by the time H1.

In the next step S203, the CPU 30a stores information relating to the frequency k1(n) in the auxiliary memory unit 30c.

In the next step S204, a determination is made as to whether or not the counter value n is larger than 3. When the determination in step S204 is negative (step S204: No), the CPU 30a moves to the next step S205, increments the counter value n by 1 and then returns to step S202. Following this, the CPU 30a repeatedly executes the processes in step S202 through S205 until the determination in step S204 is affirmative. Through this, information relating to the blink frequencies per unit time k1(1), k1(2), k1(3) and k1(4) are stored in the auxiliary memory unit 30c each time the time H1 elapses.

On the other hand, when the determination in step S204 is affirmative, the CPU 30a moves to step S208.

In the next step S208, the CPU 30a sets the average value $k_{AVG}$ of the frequencies k1(1), k1(2), k1(3) and k1(4) as the reference value S.

In the next step S211, the CPU 30a counts the frequency C2(j) with which the driver 100 blinked during the interval from the present time until the time H2 has elapsed, with reference to the image information D(m). Furthermore, the frequency C2(j) is divided by the time H2 to detect the blink frequency k2(j) per unit time.

In the next step S212, the CPU 30a stores information relating to the frequency k2(j) in the auxiliary memory unit 30c.

In the next step S213, the CPU 30a determines whether or not the frequency k2(j) is larger than a value found by adding the correction value $\alpha$ to the reference value S. When the determination in step S213 is negative (step S213: No), the CPU 30a returns to step S211. Furthermore, the processes from step S211 to S213 are repeatedly executed until the determination in step S213 is affirmative.

On the other hand, when the determination in step S213 is affirmative (step S213: Yes), the CPU 30a moves to step S214.

In step S214, the CPU 30a determines that the status of the driver 100 is an initial state when alertness is declining, and outputs the determination results to an external device and/or the like.

As explained above, with this preferred embodiment while a prescribed time is elapsing from a time t1 when the vehicle's ignition switch is turned on, a reference value S for detecting an initial state is set. Consequently, this reference value S becomes an indicator accurately indicating to a certain degree that the driver is in a state of high alertness.

With this preferred embodiment, by comparing this reference value S and the blink frequency k2(j) that is an indicator reflecting the driver's current status, a determination is made as to whether or not the driver is in a state of high alertness. Accordingly, it is possible to accurately determine whether or not the driver is in a state of high alertness.

With this preferred embodiment, the driver's alertness is determined to be high when the driver's blink frequency per unit time k2(j) exceeds the reference value S. Accordingly, it is possible to accurately detect the initial state when the driver's alertness declines.

Above, the preferred embodiments of the present invention were described, but the present invention is not limited by the above-described preferred embodiments.

For example, with the above-described second preferred embodiment, a determination as to whether or not the driver 100 is in an alert state was made by detecting the frequency D with which the facial orientation of the driver 100 changed, and comparing this frequency D with a threshold value. This is intended to be illustrative and not limiting, for it would be fine to make a determination as to whether or not the driver 100 is in an alert state by detecting a frequency D2 with which the orientation of the line of sight of the driver 100 changed, and comparing this frequency D2 and a threshold value.

With the above-described preferred embodiments, the reference value S was set based on image information output from immediately after the driver turned the ignition switch on until a prescribed time had elapsed. This is intended to be illustrative and not limiting, for it would also be fine, for example, to determine that the alertness of the driver is high when the degree of change in the orientation of the driver's face or the orientation of the driver's line of sight is large, to count the driver's blink frequency and to set the reference value S based on this count result. When the degree of change in the orientation of the driver's face or the orientation of the line of sight is large, in general the driver could be said to be in a state of high alertness. Accordingly, the reference value S computed based on blink frequency when the degree of change in the orientation of the driver's face or the orientation of the line of sight is large is an indicator accurately indicating to a certain degree that the driver is in a state of high alertness.

The threshold values in steps S 104, S 106 and S204 in the second preferred embodiment and the third preferred embodiments are examples, and the present invention is not limited to these.

The functions of the alertness determination device 30 according to the above-described preferred embodiments can be realized by special hardware or can be realized by an ordinary computer system.

The program recorded in the auxiliary memory unit 30c of the alertness determination device 30 in the second preferred embodiment and the third preferred embodiment may constitute a device that executes the above-described processes by the program being stored and distributed on a computer-readable recording medium such as a flexible disk, a CD-ROM (Compact Disk Read-Only Memory), a DVD (Digital Versatile Disk), an MO (Magneto-Optical Disk) and/or the like, and the program being installed on a computer.

In addition, the program may be stored on a disk device and/or the like possessed by a prescribed server device on a communications network such as the Internet, and for example may be downloaded to a computer by being overlaid on carrier waves.

With the above-described preferred embodiments, the reference value S is set based on the driver's blink frequency per unit time. This is one example, and it would also be fine for the reference value S to be set based on other factors. Below, specific examples of this are explained simply.

In setting the reference value, it is conceivable that the openness of the driver's eyelids could be used.

For example, the openness of the driver's eyelids in an alert state could be measured. Furthermore, the average value of the openness of the eyelids per unit time may be computed, excluding times when the driver is blinking, and that average value may be set as the reference value. In this case, if the average value of the openness per unit time falls below the reference value, it can be determined that the driver's state is an initial state when alertness is starting to decline.

In setting the reference value, it is conceivable that the elapsed time when the diver's eyelids are closed (closed-eye time) could be used. For example, the closed-eye time of a driver in an alert state could be measured. Furthermore, the average value of the closed-eye time per unit time may be computed, and this average value may be set as the reference value. In this case, when the closed-eye time per unit time climbs above the reference value, it can be determined that the driver's state is an initial state when alertness is starting to decline.

In addition, when setting the reference value using closed-eye time, after setting the sum of the closed-eye time in a prescribed time as the reference value, when the sum of the close-eye time in the prescribed time climbs above the reference value it can be determined that the driver's state is an initial state when alertness is starting to decline.

In setting the reference value, it is conceivable that the orientation of the driver's face could be used. For example, the orientation or angle (Euler angle roll) of the face of the driver in an alert state could be measured. Furthermore, the standard deviation of the orientation of the face per unit time may be computed and this standard deviation may be set as the reference value. In this case, when the standard deviation of the orientation of the driver's face per unit time climbs above the reference value, it can be determined that the driver's state is an initial state when alertness is starting to decline.

In addition, in setting the reference value it would be fine to use, besides the Euler angle roll for facial orientation, the Euler angle pitch or yaw.

In setting the reference value, it is conceivable that the orientation of the driver's line of sight could be used. For example, the orientation or angle (Euler angle roll) of the line of sight of the driver in an alert state could be measured. Furthermore, the standard deviation of the orientation of the line of sight per unit time may be computed and this standard deviation may be used as the reference value. In this case, when the standard deviation of the orientation of the driver's line of sight per unit time falls below the reference value, it can be determined that the driver's state is an initial state when alertness is starting to decline.

In addition, in setting the reference value it would be fine to use, besides the Euler angle roll for orientation or line of sight, the Euler angle pitch or yaw.

In setting the reference value, it is conceivable that the displacement in the sideways direction of the vehicle the driver is driving could be used. For example, the displacement in the sideways direction of the vehicle being driven by the driver in an alert state could be measured. Furthermore, the standard deviation of the displacement per unit time may be found and this standard deviation may be used as the reference value. In this case, when the standard deviation of the displacement in the sideways direction per unit time climbs above the reference value, it can be determined that the driver's state is an initial state when alertness is starting to decline. It is conceivable that the displacement of the vehicle in the sideways direction could be computed by integrating outputs from acceleration sensors.

In setting the reference value, it is conceivable that the driver's pulse could be used. For example, the pulse of a driver in an alert state could be measured. Furthermore, the average value of the pulse per unit time may be computed and this average value may be set as the reference value. In this case, when the average value of the pulse per unit time falls below the reference value, it can be determined that the driver's state is an initial state when alertness is starting to decline.

In addition, the pulse may be converted to a prescribed indicator, and the determination of the driver's state can be made based on this indicator.

For example, when the ratio (N2/N1) of the number N2 by which the RR interval of the pulse is greater than a prescribed value (48ms) to the pulse N1 in a prescribed time exceeds the reference value, it can be determined that the driver's state is an initial state when alertness is starting to decline.

In addition, when the sum of the squares of the differences of the RR interval per unit time exceeds the reference value, it may be determined that the driver's state is an initial state when alertness is starting to decline.

In addition, when the power value PL of a specific frequency range (for example, 0.04 to 0.15 Hz) of RR interval changes, or the power value PH of a specific frequency range (for example 0.15 to 0.4) of RR interval changes exceeds the reference value, it may be determined that the driver's state is an initial state when alertness is starting to decline.

Furthermore, when the ratio of the power value PL to the power value PH (PF/PH), or the ratio of the power value PL to the sum of the power values PL and PH (PL/(PH+PL)) exceeds the reference value, it may be determined that the driver's state is an initial state when alertness is starting to decline.

In setting the reference value, it is conceivable that the driver's respiration interval could be used. For example, the respiration interval of a driver in an alert state could be measured. Furthermore, the average value of the respiration intervals per unit time may be computed and this average value may be set as the reference value. In this case, when the average value of the respiration intervals per unit time climbs above the reference value, it can be determined that the driver's state is an initial state when alertness is starting to decline.

In addition, when respiration intervals are used in setting the reference value, after the standard deviation in respiration intervals per unit time is set as the reference value, when the standard deviation in respiration intervals per unit time climbs above the reference value, it may be determined that the driver's state is an initial state when alertness is starting to decline.

Having described and illustrated the principles of this application by reference to one or more preferred embodiments, it should be apparent that the preferred embodiments may be modified in arrangement and detail without departing from the principles disclosed herein and that it is intended that the application be construed as including all such modifications and variations insofar as they come within the spirit and scope of the subj ect matter disclosed herein.

This application claims the benefit of Japanese Patent Application 2010-67001, filed 23 March 2010, the entire disclosure of which is incorporated by reference herein.

Industrial Applicability

The alertness determination device, alertness determination method and program of the present invention are suitable for determining a driver's alertness.

Description of Numerical References

10 Alertness determination system

20 Photography device

30 Alertness determination device

30a CPU

30b Main memory unit

30c Auxiliary memory unit

30d Display unit

30e Input unit

30f Interface unit

30g System bus

31 Memory unit

32 Face orientation detection unit

33 Line-of-sight orientation detection unit

34 First counter

35 Alertness determination unit

36 Reference value determination unit

37 Second counter

38 Initial state determination unit

100 Driver

F Face

IM Image

**Claims**

1. An alertness determination device for determining the alertness of a driver driving a vehicle, the alertness determination device comprising:

   a first count means for counting the frequency with which the driver blinks based on images obtained by photographing the driver, when the driver is in a prescribed state;
   a reference value setting means for setting a reference value based on the count results of the first count means;
   a second count means for counting the frequency with which the driver blinks within a prescribed time based on images obtained by photographing the driver, after the reference value is set; and
   a determination means for determining that the alertness of the driver is declining when the frequency counted by the second count means exceeds the reference value.

2. The alertness determination device of Claim 1, wherein the prescribed state is a state in which the frequency with which the orientation of the face of the driver changes within a given time exceeds a prescribed threshold value.

3. The alertness determination device of Claim 1, wherein the prescribed state is a state in which the frequency with which the orientation of the line of sight of the driver changes within a given time exceeds a prescribed threshold value.

4. The alertness determination device of Claim 1, wherein the prescribed state is a state from when the driver begins driving until a prescribed time has elapsed.

5. The alertness determination device of any of Claims 1 to 4, further comprising:

a detecting means for detecting the orientation of the face of the driver from the images; wherein the first count means counts the frequency with which the driver blinks when the orientation of the face of the driver detected by the detecting means is substantially equivalent to the direction of progress of the vehicle.

6. The alertness determination device of Claim 5, wherein the second count means counts the frequency with which the driver blinks when the orientation of the face of the driver detected by the detecting means is substantially equivalent to the direction of progress of the vehicle.

7. An alertness determination method for determining the alertness of a driver driving a vehicle, the alertness determination method comprising:

a first procedure for counting the frequency with which the driver blinks based on images obtained by photographing the driver, when the driver is in a prescribed state;
a second procedure for setting a reference value based on the count results of the first procedure;
a third procedure for counting the frequency with which the driver blinks within a prescribed time based on images obtained by photographing the driver, after the reference value is set; and
a fourth procedure for determining that the alertness of the driver is declining when the frequency counted in the third procedure exceeds the reference value.

8. A program for causing a computer to function as:

a first count means for counting the frequency with which the driver blinks based on images obtained by photographing the driver, when the driver is in a prescribed state;
a reference value setting means for setting a reference value based on the count results of the first count means;
a second count means for counting the frequency with which the driver blinks within a prescribed time based on images obtained by photographing the driver, after the reference value is set; and
a determination means for determining that the alertness of the driver is declining when the frequency counted by the second count means exceeds the reference value.

# FIG.1

PHOTOGRAPHY DEVICE — 20

30

FACE ORIENTATION
DETECTION UNIT — 32

LINE-OF-SIGHT ORIENTATION
DETECTION UNIT — 33

FIRST COUNTER — 34

31

MEMORY
UNIT

ALERTNESS DETERMINATION
DEVICE — 35

REFERENCE VALUE SETTING
UNIT — 36

SECOND COUNTER — 37

INITIAL STATE
DETERMINATION UNIT — 38

DETERMINATION
RESULTS

# FIG.2

# FIG.3

DETERMINATION
RESULTS

FIG.4

START

RESET COUNTER VALUE n
TO n=1 ～S101

DETECT FACE ORIENTATION,
BLINK FREQUENCY ～S102

STORE DETECTED DATA ～S103

S104
n > 2?
No
Yes

S105
n←n+1

S106
n < 10?
Yes
No

S107
ALERT STATE?
No
Yes

S108
SET REFERENCE VALUE

S109
SET REFERENCE VALUE
BASED ON ALL DATA

DETECT BLINK FREQUENCY ～S111

STORE DETECTED DATA ～S112

S113
BLINK FREQUENCY >
REFERENCE VALUE + $\alpha$ ?
No
Yes

OUTPUT DETERMINATION
RESULTS ～S114

END

# FIG.5

# FIG.6

```
        START
          │
          ▼
┌──────────────────────┐
│ RESET COUNTER VALUE n │ ～ S201
│      TO n=1           │
└──────────────────────┘
          │
          ▼ ◄─────────────┐
┌──────────────────────┐  │
│ DETECT FACE ORIENTATION, │ ～ S202
│   BLINK FREQUENCY     │  │
└──────────────────────┘  │
          │               │
          ▼               │   S205
┌──────────────────────┐  │  ┌─────────┐
│  STORE DETECTED DATA  │ ～ S203  │ n←n+1 │
└──────────────────────┘  │  └─────────┘
          │               │       ▲
          ▼               │       │
       ╱╲                 │       │
      ╱  ╲    n > 3?  ～ S204      │
     ╱    ╲              No───────┘
      ╲  ╱
       ╲╱
      Yes│
          ▼
┌──────────────────────┐
│  SET REFERENCE VALUE  │ ～ S208
└──────────────────────┘
          │
          ▼ ◄─────────────┐
┌──────────────────────┐  │
│ DETECT BLINK FREQUENCY │ ～ S211
└──────────────────────┘  │
          │               │
          ▼               │
┌──────────────────────┐  │
│  STORE DETECTED DATA  │ ～ S212
└──────────────────────┘  │
          │               │
          ▼               │
       ╱╲                 │
      ╱  ╲  BLINK FREQUENCY >   ～ S213
     ╱    ╲ REFERENCE VALUE + α ?
      ╲  ╱         No────────┘
       ╲╱
      Yes│
          ▼
┌──────────────────────┐
│ OUTPUT DETERMINATION  │ ～ S214
│      RESULTS          │
└──────────────────────┘
          │
          ▼
        END
```

## INTERNATIONAL SEARCH REPORT

| International application No. |
|---|
| PCT/JP2011/055547 |

A.  CLASSIFICATION OF SUBJECT MATTER
*A61B5/18*(2006.01)i, *B60K28/06*(2006.01)i, *B60R11/04*(2006.01)i, *B60W40/08*(2006.01)i, *G08G1/16*(2006.01)i

According to International Patent Classification (IPC) or to both national classification and IPC

B.  FIELDS SEARCHED

Minimum documentation searched (classification system followed by classification symbols)
A61B5/18, B60K28/06, B60R11/04, B60W40/08, G08G1/16

Documentation searched other than minimum documentation to the extent that such documents are included in the fields searched
Jitsuyo Shinan Koho        1922–1996    Jitsuyo Shinan Toroku Koho    1996–2011
Kokai Jitsuyo Shinan Koho  1971–2011    Toroku Jitsuyo Shinan Koho    1994–2011

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y/A | JP 2008-186263 A  (Denso Corp.), 14 August 2008 (14.08.2008), (Family: none) | 1-4,7,8/5,6 |
| Y/A | JP 2005-257470 A  (Toyota Motor Corp.), 22 September 2005 (22.09.2005), (Family: none) | 1-4,7,8/5,6 |
| Y/A | JP 2008-161664 A  (Toyota Motor Corp.), 17 July 2008 (17.07.2008), & US 2010/0049066 A      & EP 2092889 A1 & WO 2008/069337 A1      & KR 10-2009-0089841 A & CN 101547643 A | 2,3/1,4-8 |
| A | JP 2003-571 A (Mitsubishi Electric Corp.) 07 January 2003 (07.01.2003), (Family: none) | 1-8 |

☐  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 March, 2011 (29.03.11) | 12 April, 2011 (12.04.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006109980 A

- JP 2010067001 A